# FASCICULE DE BREVET EUROPEEN

(11) **EP 1 989 996 B1**
(45) Date de publication et mention de la délivrance du brevet: **20.07.2011**
(21) Numéro de dépôt: 08156010.4
(22) Date de dépôt: 09.05.2008
(51) Int. Cl.: A61B 1/05

(54) **Exploration d'une cavité avec capteur d'image**
Untersuchung eines Hohlraums mit einem Bildsensor
Exploration of a cavity with an image sensor

(30) Priorité: 10.05.2007 FR 0703344; 10.05.2007 FR 0703345
(43) Date de publication de la demande: 12.11.2008
(73) Titulaire: STMicroelectronics SA, 92120 Montrouge (FR)
(72) Inventeur: Luneau, Dominique, 74270 Chilly (FR); Varillon, Paul, 38100, Grenoble (FR)
(74) Mandataire: Cabinet Plasseraud

(56) Documents cités:
- EP-A- 1 455 216
- US-A- 4 832 003
- US-A- 5 021 888

## Description

La présente invention se rapporte à l'exploration de cavités, plus précisément à l'exploration de cavités de petites dimensions comprenant des zones qui ne sont pas directement visibles à l'oeil nu.

Une telle exploration peut se pratiquer à l'aide d'un instrument comportant un système optique et une source lumineuse qui permettent de fournir des images de la zone considérée. Un tel instrument peut comprendre un endoscope.

Il comprend classiquement une tête, ou tête d'exploration, qui correspond à la partie destinée à être introduite dans une cavité à explorer et à fournir des données depuis cette cavité vers un module extérieur destiné à gérer l'interface avec un utilisateur du dispositif d'exploration pour lui fournir des images de la cavité.

On connaît ainsi un dispositif d'exploration qui consiste en une pluralité de fibres optiques destinées à être introduites dans la cavité à explorer afin de transmettre des signaux optiques depuis la zone explorée vers un module qui, lui, est destiné à rester à l'extérieur de la cavité. Dans ce contexte, le module extérieur comprend un capteur d'image qui est en charge de capter les signaux optiques transmis par les fibres optiques. Dans une telle architecture, les signaux optiques transmis depuis l'intérieur de la cavité vers l'extérieur de la cavité sont donc traitées à l'extérieur de la cavité, au niveau du module extérieur au capteur d'image.

Il convient de noter qu'un tel dispositif d'exploration présente un coût important du fait de la présence des fibres optiques reliant la tête d'exploration au capteur d'image. Il en résulte que ce dispositif ne peut raisonnablement pas être un dispositif jetable, c'est-à-dire à usage unique.

De ce fait, il est requis de procéder à une désinfection du matériel, et au moins des fibres optiques, entre deux utilisations du dispositif d'exploration, chaque désinfection présentant également, en soi, un coût important.

En outre, la transmission des signaux optiques par les fibres optiques jusqu'au capteur d'image situé dans le module extérieur peut dégrader la qualité des images obtenues au niveau du module extérieur.

On connait également un dispositif d'exploration, tel que celui décrit dans le document US 6,939,295, qui se présente sous une forme de capsule adaptée pour être introduite dans la cavité à explorer. Une telle capsule comprend un capteur CMOS couplé à une entité d'émission radio apte à envoyer des informations captées depuis l'intérieur de la cavité à explorer vers l'extérieur pour fournir des images de la cavité à un utilisateur. Afin que le capteur CMOS soit de petite taille, il est prévu d'utiliser un support de circuit intégré flexible. Une telle architecture est ainsi basée sur une technologie qui induit un coût de fabrication important d'un tel dispositif d'exploration.

Le document EP 1 400 214 décrit également un dispositif d'exploration de cavité qui comprend une partie d'exploration destinée à être introduite dans la cavité à explorer et qui comprend un capteur d'image de type CMOS (en anglais pour 'Complementary Metal Oxide Semi-conducteur'). Un logement dédié à ce capteur d'image est prévu dans la partie d'exploration. Dans une telle architecture le circuit intégré comprenant le capteur d'image CMOS ainsi que les connexions qui le relie à un module extérieur, prend de la place dans la partie d'exploration. En effet, classiquement, lorsqu'on souhaite relier un fil à une pastille conductrice d'un support de circuit intégré via une connexion mécanique, on utilise des connecteurs male et femelle pour que cette connexion présente une importante résistance mécanique. Un de ces connecteurs est soudé sur le circuit intégré, l'autre connecteur étant serti au fil. Puis, le fil, ou micro-câble, est relié au circuit intégré par la connexion entre les connecteurs male et femelle. Or, une telle connexion est relativement volumineuse. De ce fait, le logement du circuit intégré est volumineux.

Or, le gain de place est un facteur important pour l'exploration de cavité de taille réduite.

EP 1 455 216 A1, US 4, 832, 003 et US 5,021,888 décrivent d'autres dispositifs d'exploration faisant partie de l'état de la technique le plus proche.

La présente invention vise à améliorer la situation.

L'invention est définie par les revendications annexées.

Un premier aspect de la présente invention propose un procédé de fabrication d'une tête de dispositif d'exploration d'une cavité, cette tête comprenant un support de circuit intégré présentant des première et seconde faces et une pluralité d'orifices traversant, à chaque orifice étant associées des première et seconde pastilles conductrices respectivement positionnées sur les première et seconde faces du circuit intégré.

Le procédé comprend les étapes suivantes mises en oeuvre pour chaque orifice support de circuit intégré :
/a/ positionner un micro-câble conducteur respectif dans l'orifice traversant, ce micro-câble présentant une partie dénudée sur une longueur supérieure ou égale à l'épaisseur du support;
/b/ souder le micro-câble sur les première et seconde pastilles conductrices associées ;
/c/ coller le micro-câble avec une colle sur les première et seconde pastilles conductrices associées ; et
/d/ mouler dans des première et seconde couches de résine le micro-câble respectivement sur les première et seconde pastilles conductrices, ces couches de résine couvrant complètement la partie dénudée du micro-câble.

Grâce à ces dispositions, il est possible de créer des connexions mécaniques résistantes tout en manipulant un circuit intégré et des micro-câbles de très petites dimensions.

Dans ce contexte, on entend par 'micro-câble' un câble fin, ou encore fil, présentant un diamètre relativement réduit. On peut notamment prévoir d'utiliser un micro-câble présentant un AGWG 36 (pour 'American Wire Gage' en anglais). Un tel micro-câble peut présenter un diamètre sensiblement égal à 0.15 mm. On peut ainsi obtenir une tête de dispositif d'exploration qui est pourvue d'un capteur d'image, de type CMOS par exemple, réalisé sur le support de circuit intégré et qui peut être introduite dans de petites cavités en étant relié vers l'extérieur par l'intermédiaire des micro-câbles.

En procédant selon les étapes énoncées ci-dessus dans un mode de réalisation de la présente invention, il n'est plus requis de créer des connexions mécaniques, telles qu'énoncées ci-avant, via des connecteurs male et femelle qui prennent de la place et ne permettent de ce fait pas d'obtenir un circuit intégré de petite taille.

En obtenant un circuit intégré de petite dimension selon un mode de réalisation de la présente invention, il n'est plus requis non plus d'utiliser un support de circuit intégré flexible, du type de celui qui est utilisé dans la capsule du document US 6,939,295, dont le coût est important. En effet, dans un mode de réalisation de la présente invention, la réduction de taille du circuit intégré et de ses connexions mécaniques, est obtenue par la mise en place de connexions telles que décrites ici.

En outre, en permettant d'introduire directement le capteur dans la cavité à explorer, des fibres optiques ne sont plus requises. Une telle caractéristique permet de réduire le coût d'un dispositif d'exploration du type endoscope et de pouvoir de jeter la partie destinée à être introduite dans la cavité à explorer, après une unique utilisation. Cette caractéristique permet alors d'éviter le coût de désinfection de ce dispositif, qui peut être très important, tout en garantissant une bonne qualité d'images.

De plus, le support de circuit intégré peut recevoir un circuit intégré comprenant un capteur des images de la cavité à explorer qui peuvent alors être captées directement. Les signaux optiques ne sont de ce fait pas transmis via des fibres optiques. Une telle prise directe des images permet d'obtenir une meilleure qualité d'image.

Grâce aux dispositions de connexions mécaniques du support de circuit intégré, on peut avantageusement utiliser un capteur d'image de type CMOS, dont le coût est moins élevé qu'un capteur d'image de type CCD (en anglais pour 'Charge-Coupled Device') par exemple. En effet, un capteur d'image de type CCD présente une taille qui est peut être plus petite que celle présentée par un capteur CMOS et de ce fait, l'utilisation d'un capteur d'image CCD peut permettre plus facilement d'obtenir un circuit intégré de petite taille. Mais, l'utilisation de la technologie des capteurs CCD induit un coût plus important que celle des capteurs CMOS. En revanche, l'utilisation de la technologie CMOS permet de prévoir une tête d'exploration jetable.

De telles connexions, selon un mode de réalisation de la présente invention, permettent donc de libérer de la place sur le support du circuit intégré à côté du capteur d'image, de telle sorte qu'il est alors avantageusement possible de prévoir un emplacement pour une source de lumière, par exemple de type diode électroluminescente.

Le circuit intégré peut comprendre au moins une diode électroluminescente et ainsi permettre l'exploration de la cavité en éclairant la zone pour laquelle on souhaite obtenir des images.

Comme les connexions des micro-câbles sur le circuit intégré permettent un gain de place sur le support du circuit intégré, on peut avantageusement prévoir également, dans un mode de réalisation de la présente invention, que le support présente un orifice adapté pour recevoir un outil d'intervention dans la cavité et/ou un orifice adapté pour recevoir un canal d'acheminement d'eau.

On peut avantageusement prévoir à cet effet que le support du circuit intégré présente une forme arrondie telle qu'un cercle permettant une répartition pertinente du capteur d'image, des sources de lumière et des orifices destinés à recevoir le canal d'acheminement d'eau et les outils.

Dans un mode de réalisation de la présente invention, la tête présente une première et une seconde face d'exploration et comprend en outre un autre support de circuit intégré par application des étapes /a/ à /d/, lesdits supports de circuit intégré comprenant respectivement un premier et un second capteur d'image ; ledit procédé de fabrication comprenant les étapes suivantes :
- disposer le support de circuit intégré du premier capteur d'image sur la première face d'exploration adaptée pour capter des images d'une première région de la cavité ;
- disposer le support de circuit intégré du second capteur d'image sur la seconde face d'exploration adaptée pour capter des images d'une seconde région de la cavité ;
lesdites première et seconde régions de la cavité étant distinctes l'une de l'autre.

Grâce à ces dispositions, cette tête permet de capter de manière simultanée des images de deux zones différentes de la cavité à explorer. L'utilisateur n'est de ce fait pas confronté au problème de l'orientation de la tête d'exploration, notamment lors de l'exploration d'une cavité très étroite, tel qu'il peut se poser au cours de l'utilisation d'une tête d'exploration pourvue d'un seul capteur d'image.

En outre, cet utilisateur est en mesure de visualiser de manière simultanée les images de deux zones distinctes de la cavité, l'une pouvant ainsi lui servir pour se repérer lors d'un déplacement de la tête dans la cavité, l'autre lui permettant de guider ses outils, le cas échéant, sur une zone d'intervention.

On peut alors prévoir que les deux capteurs d'image sont reliés à un terminal extérieur, ou dispositif d'interface, de telle sorte que les images captées par ces deux capteurs d'image sont transmises depuis la tête d'exploration jusqu'à ce terminal. L'utilisateur peut alors visualiser simultanément, par exemple dans deux fenêtres affichées sur l'écran du terminal, d'une part, le flux d'images captées de la première région de la cavité et, d'autre part, le flux d'images captées de la seconde région de la cavité.

Ainsi, l'utilisateur peut déplacer la tête d'exploration en se référant aux images affichées sur une des fenêtres et il peut intervenir sur la région de la cavité à laquelle se rapportent les images affichées sur l'autre fenêtre.

La première face d'exploration de la tête de dispositif d'exploration peut correspondre à une face latérale de cette tête et la seconde face d'exploration peut correspondre à une face avant de cette tête.

Un deuxième aspect de la présente invention propose une tête de dispositif d'exploration d'une cavité, ladite tête comprenant un support de circuit intégré présentant des première et seconde faces et une pluralité d'orifices traversants, à chaque orifice étant associées des première et seconde pastilles conductrices respectivement positionnées sur les première et seconde faces du support de circuit intégré, dans laquelle, à chaque orifice traversant dudit support de circuit intégré correspond un micro-câble présentant une partie dénudée sur une longueur supérieure ou égale à l'épaisseur dudit support, ledit micro-câble étant soudé et collé sur les première et seconde pastilles conductrices associées, et moulé dans des première et seconde couches de résine le micro-câble respectivement sur les première et seconde pastilles conductrices, lesdites couches de résine couvrant complètement la partie dénudée dudit micro-câble.

Dans un mode de réalisation de la présente invention, la tête de dispositif d'exploration présente des première et seconde faces d'exploration et comprend en outre un autre support de circuit intégré ; lesdits supports de circuit intégré comprenant respectivement un premier et un second capteur d'image ;
le premier capteur d'image étant disposé sur la première face d'exploration et adapté pour capter des images d'une première région de la cavité ; et le second capteur d'image étant disposé sur la seconde face d'exploration et adapté pour capter des images d'une seconde région de la cavité ;
lesdites première et seconde régions de la cavité étant distinctes l'une de l'autre.

La première face d'exploration peut correspondre à une face latérale de la tête de dispositif d'exploration et la seconde face d'exploration peut, elle, correspondre à une face avant de ladite tête.

Un troisième aspect de la présente invention propose un dispositif d'exploration d'une cavité comprenant :
- une tête de dispositif d'exploration d'une cavité, ladite tête étant, d'une part, pourvue de micro-câbles par application d'un procédé de fabrication selon le premier aspect de la présente invention, et, d'autre part, comprenant au moins un capteur d'image; et
- un module d'interface adapté pour fournir des images captées par ledit capteur d'image, ledit module d'interface étant relié à la tête de dispositif d'exploration par lesdits micro-câbles.

La tête peut présenter un orifice adapté pour recevoir un canal d'eau, et un orifice adapté pour recevoir un outil d'intervention dans la cavité.

D'autres aspects, buts et avantages de l'invention apparaîtront à la lecture de la description d'un de ses modes de réalisation.

L'invention sera également mieux comprise à l'aide des dessins, sur lesquels ::
- La figure 1 illustre un dispositif d'exploration selon un mode de réalisation de la présente invention ;
- les figures 2-A et 2-B illustrent respectivement une vue de face et une vue de dos d'une tête d'un dispositif d'exploration selon un mode de réalisation de la présente invention ; et
- la figure 3 illustre un circuit intégré d'une tête d'un dispositif d'exploration selon un mode de réalisation de la présente invention dans un premier et un second cas ;
- la figure 4 illustre les principales étapes d'un procédé de fabrication s'une tête de dispositif d'exploration selon un mode de réalisation de la présente invention ;
- la figure 5 illustre une connexion d'un micro-câble avec le support de circuit intégré dans une tête fabriquée selon un mode de réalisation de la présente invention ;
- la figure 6 illustre une tête de dispositif d'exploration selon un mode de réalisation de la présente invention ; et
- la figure 7 illustre un circuit intégré d'une tête d'exploration d'un dispositif d'exploration selon un mode de réalisation de la présente invention.

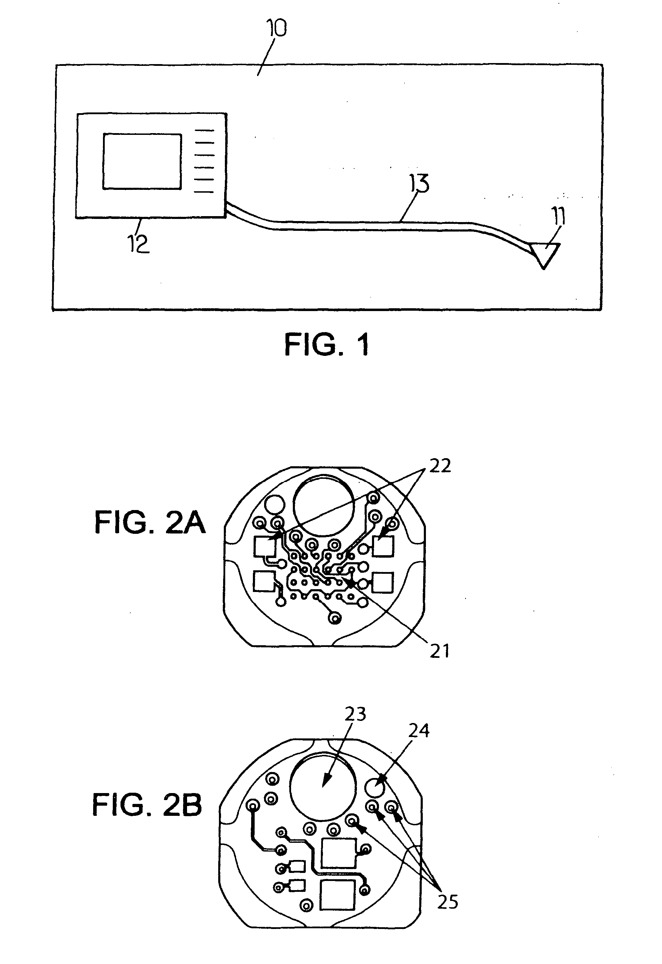

La figure 1 illustre un dispositif d'exploration selon un mode de réalisation de la présente invention.

Un tel dispositif peut être utilisé avantageusement pour l'exploration de toute cavité qui n'est pas visible directement par l'oeil humain, et notamment des cavités relativement étroites et difficiles d'accès, puisque ce dispositif d'exploration présente une tête de petite dimension.

Aucune limitation n'est attachée à la présente invention au regard du domaine de l'utilisation d'un tel dispositif d'exploration. Il peut aussi bien être utilisé dans le domaine médical, ou le domaine de l'avionique, ou encore celui de l'aéronautique ...

De plus, cette tête comprenant un capteur d'image permet de fournir à un utilisateur du dispositif d'exploration des images de bonne qualité.

Un tel dispositif 10 comprend une tête 11 d'exploration d'une cavité qui est reliée à un module d'interface 12 via des micro-câbles 13. Dans un mode de réalisation de la présente invention, cette tête comprend un capteur d'image de type CMOS.

Aucune limitation n'est attachée à la présente invention au regard du module d'interface. Un tel module d'interface est adapté pour recevoir des données relatives à des images captées par la tête d'exploration, plus précisément par le capteur d'image, et pour les traiter de manière à les fournir sous une forme exploitable par l'utilisateur du dispositif d'exploration. Ce module d'interface peut être par exemple un ordinateur.

Les figures 2-A et 2-B illustrent respectivement une vue de face et une vue de dos d'une tête d'un dispositif d'exploration selon un mode de réalisation de la présente invention. Il convient de noter que les connexions des micro-câbles selon un mode de réalisation de la présente invention permettent avantageusement d'utiliser le support du circuit intégré supportant le capteur d'image CMOS pour introduire au moins une source de lumière et des guides pour un canal acheminant de l'eau depuis l'extérieur de la cavité jusque dans la cavité et pour un canal correspondant à un outil d'intervention dans la cavité. De tels guides correspondent, dans cet exemple, à un premier et un second orifice.

La figure 2-A illustre le support de circuit intégré comprenant un capteur d'image CMOS 21 et deux diodes électroluminescentes 22.

La figure 2-B illustre un orifice 23 traversant le support de circuit intégré pour recevoir un outil d'intervention dans la cavité à explorer. Ainsi, l'utilisateur du dispositif d'exploration est en mesure d'effectuer certaines opérations dans la cavité en cours d'exploration. Un autre orifice 24 adapté pour recevoir un canal d'acheminement en eau est également prévu sur le support de circuit intégré. Le support de circuit intégré présente des pastilles conductrices 25.

Il convient de noter que le procédé de fabrication de la tête d'exploration selon un mode de réalisation de la présente invention permet un gain de place tel qu'il est ainsi possible de prévoir directement dans le support de circuit intégré des orifices qui peuvent notamment être adaptés pour amener de l'eau jusque dans la cavité et pour recevoir un outil d'intervention sur la cavité.

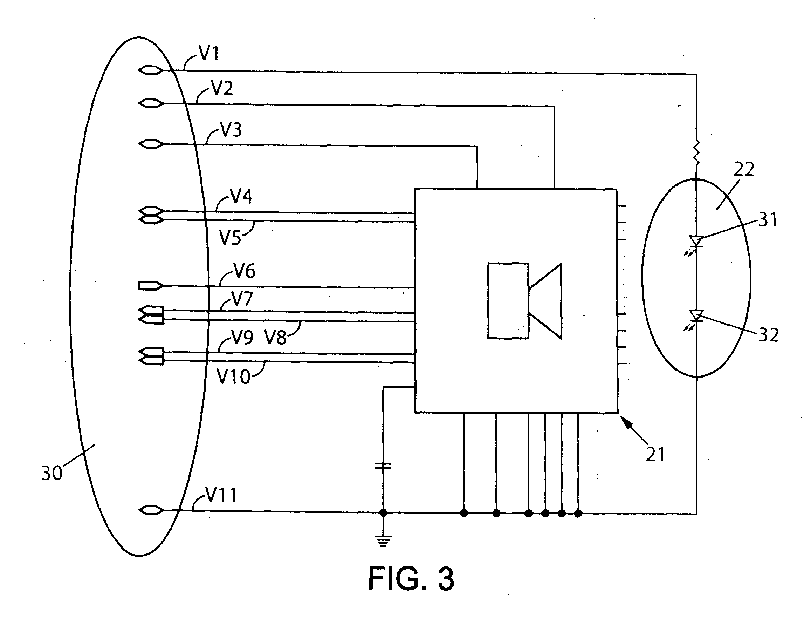

La figure 3 illustre en détail le circuit intégré compris dans la tête d'exploration selon un mode de réalisation de la présente invention.

Ce circuit intégré comprend les deux diodes électroluminescentes 22, référencées respectivement 31, 32. Il comprend également le capteur d'image CMOS 21. Enfin, des entrées et sorties de ces éléments, diodes électroluminescentes et capteur d'image, sont reliées via des micro-câbles au module d'interface 12.

Dans ce contexte, il est prévu de fournir l'alimentation des composants électroniques de la tête 11 par l'intermédiaire du module d'interface. Toutefois, il est aisé de prévoir d'autres configurations dans lesquelles l'alimentation est fournie via les micro-câbles 13, par un autre module que le module d'interface. Aucune limitation n'est attachée à la présente invention au regard de cet aspect.

Une zone de connexions 30 des micro-câbles est illustrée sur la figure 1. Dans ce mode de réalisation, les micro-câbles sont respectivement connectés :
- au signal d'alimentation de l'éclairage V1 ;
- aux signaux d'alimentation du capteur CMOS V2 et V3 :

- aux signaux de contrôle du capteur CMOS V4 et V5, ces signaux pouvant utiliser la norme I²C (pour *`Inter Integrated Circuit Bus'*) ;
- au signal d'horloge du capteur CMOS V6 ;
- aux signaux de transfert du flux vidéo V7, V8, V9 et V10, ces signaux pouvant utiliser la norme SubLVDS (pour 'Sub Low Voltage Differential Signalling') ;
- au signal de masse, V11.

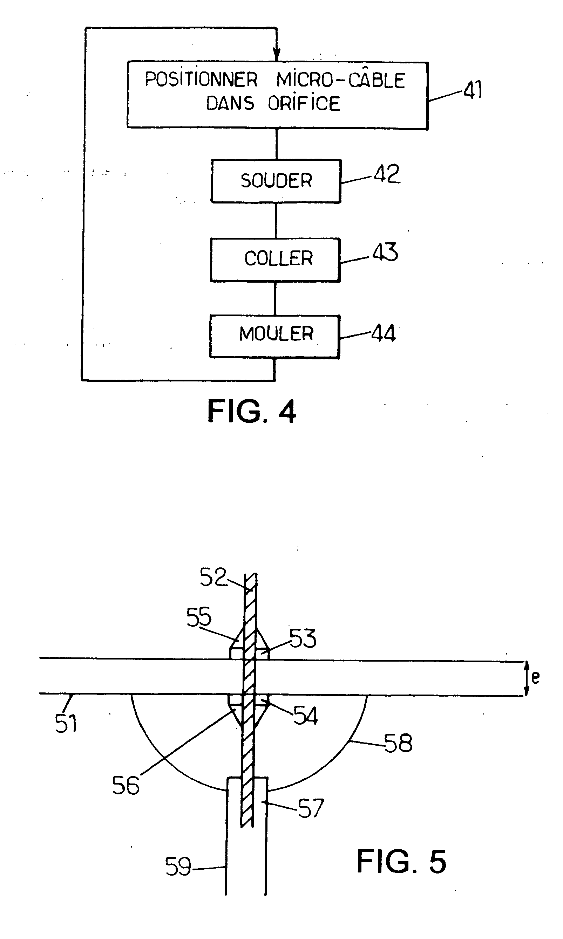

La figure 4 illustre les principales étapes du procédé de fabrication de la tête de dispositif d'exploration selon un mode de réalisation de la présente invention.

Pour fabriquer une tête d'exploration, on dispose tout d'abord d'un support pour circuit intégré. Un tel support est de préférence de forme ronde.

Sur ce support est intégré un capteur d'image CMOS, et deux diodes électroluminescentes. Ce support de circuit intégré présente un ensemble de pastilles conductrices positionnées sur le circuit intégré, par couple de pastilles conductrices situées de part et d'autre du support. Un orifice traversant le support de circuit intégré passe au travers de chacun de ces couples de pastilles conductrices.

Ces pastilles conductrices correspondent aux entrées/sorties des composants, telles que celles listées ci-avant en référence à la zone de connexions 30. Elles sont destinées à être connectées au module d'interface, ou tout au moins à un module destiné à rester à l'extérieur de la cavité et à recevoir les données relatives aux images captées par le capteur d'image de la tête d'exploration.

La figure 5 illustre une connexion effectuée selon un mode de réalisation de la présente invention. Pour effectuer ces connexions mécaniques entre, notamment, le capteur d'image et le module d'interface, on utilise un micro-câble 59 qui comprend une gaine 57 et un ou plusieurs fils 52. La présente invention ne comprend aucune limitation sur le type de micro-câble utilisé. On peut avantageusement utiliser un micro-câble présentant un diamètre d'une valeur de 0,3 mm de diamètre, par exemple.

Le micro-câble est dénudé sur une longueur qui est supérieure ou égale à l'épaisseur, référencée e, du support de circuit intégré 51. Puis, à une étape 41, le micro-câble est positionné dans l'orifice qui traverse le support de circuit intégré 51 au niveau d'un couple de pastilles conductrices 53 et 54, de telle sorte que sa partie dénudée se situe au niveau de l'épaisseur du support 51 et permet le contact du fil 52 au niveau des deux pastilles conductrices 53 et 54.

Puis, à une étape 42, le fil 52 est soudé au niveau de la pastille 53 sur la face supérieure du support du circuit intégré par une soudure 55 et au niveau de la pastille 54 sur la face inférieur du support de circuit intégré par une soudure 56.

Ensuite, à une étape 43, ce fil est en outre collé au niveau des deux pastilles conductrices 53 et 54 au moyen d'une colle qui peut être à base de résine.

Enfin, à une étape 44, l'ensemble d'une telle connexion est consolidé au moyen d'un moulage à base de résine qui est positionné au niveau des pastilles conductrices 53 et 54 de telle sorte qu'il englobe dans sa totalité, d'une part, la soudure et la colle qui ont préalablement été appliquées, et, d'autre part, la partie dénudée su micro-câble 59. Pour améliorer la résistante d'une connexion selon la présente invention, il préférable que le moulage englobe également une partie de la gaine 57 du micro-câble, comme cela est illustré à la figure 5.

De telles connexions prenant une taille réduite, il est possible de prévoir non seulement des diodes électroluminescentes sur le circuit intégré, mais en outre des orifices sur le support du circuit intégré. On peut par exemple prévoir un orifice pour le canal d'acheminement d'eau et un orifice pour un outil.

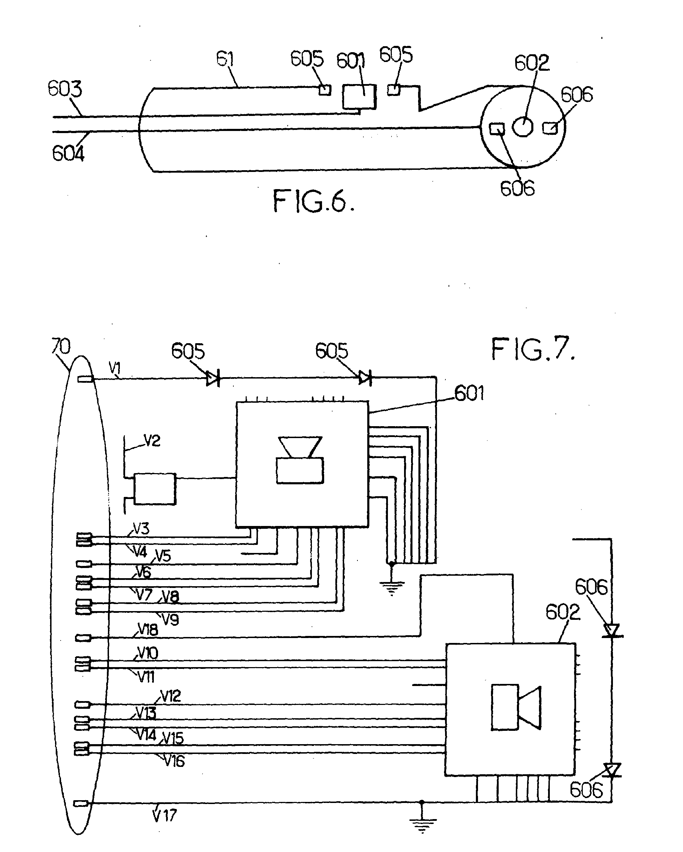

La figure 6 illustre une tête de dispositif d'exploration selon un mode de réalisation de la présente invention, cette tête 61 comprend des premier et second capteurs d'image de type CMOS (pour 'Complementary Metal Oxide Semiconductor', en anglais) 601 et 602 respectivement. Le premier capteur 601 est positionné sur une face latérale de la tête d'exploration 61, alors que le second capteur d'image est positionné sur une face avant de la tête d'exploration 61.

Dans une telle configuration, il apparait clairement que les deux capteurs d'image de la tête sont en mesure de capturer des images de deux zones complètement distinctes de la cavité à explorer.

Ces premier et second capteurs d'image sont reliés à un terminal 62 par des câbles respectifs 603 et 604 par l'intermédiaire desquels les données relatives aux deux capteurs d'image sont transmises jusqu'au terminal extérieur.

Aucune limitation n'est attachée à la présente invention au regard de l'architecture permettant de relier les capteurs d'image et le terminal. On peut notamment prévoir des dispositifs intermédiaires entre la tête d'exploration 61 et le terminal extérieur 62, qui pourraient être en charge de traiter les deux flux d'images remontant respectivement depuis les deux capteurs d'image.

En outre, il est prévu de pourvoir de sources de lumières la tête d'exploration 61 selon un mode de réalisation de la présente invention, afin d'éclairer les régions de la cavité qui sont respectivement visées par les deux capteurs d'image 601 et 602.

Ainsi, on peut prévoir deux premières sources de lumière 605, positionnées de part et d'autre du premier capteur d'image 601 sur la face latérale de la tête, ainsi que deux secondes sources de lumière 606, positionnées de part et d'autre du second capteur d'image 602 sur une face avant de la tête 61.

La figure 7 illustre un circuit intégré d'une tête d'exploration d'un dispositif d'exploration selon un mode de réalisation de la présente invention.

Ce circuit intégré comprend les deux diodes électroluminescentes 605, ainsi que les deux diodes électroluminescentes 606. Il comprend également le premier capteur d'image CMOS 601 et le second capteur d'image 602. Enfin, des entrées et sorties de ces éléments, diodes électroluminescentes et capteurs d'image, sont reliées via des micro-câbles à un module d'interface via une zone de connexion 70.

Dans ce contexte, il est prévu de fournir l'alimentation des composants électroniques de la tête 61 par l'intermédiaire du module d'interface. Toutefois, il est aisé de prévoir d'autres configurations dans lesquelles l'alimentation est fournie via la liaison 603 qui peut correspondre à des micro-câbles 603, par un autre module que le module d'interface. Aucune limitation n'est attachée à la présente invention au regard de cet aspect.

Une zone de connexions 70 des câbles est illustrée sur la figure 7. Dans ce mode de réalisation, des câbles sont respectivement connectés pour le premier capteur d'image 601 :
- au signal d'alimentation des sources de lumière V1 ;
- au signal d'alimentation du capteur d'image CMOS V2 ;
- aux signaux de contrôle du capteur d'image CMOS V3 et V4, ces signaux pouvant utiliser la norme I²C (pour *'Inter Integrated Circuit Bus'*) ;
- au signal d'horloge du capteur d'image CMOS V5 ;
- aux signaux de transfert du flux vidéo V6, V7, V8 et V9, ces signaux pouvant utiliser la norme SubLVDS (pour 'Sub Low Voltage Differential Signalling') ;
et pour le second capteur d'image 602 :
- au signal d'alimentation du capteur d'image CMOS V18 ;
- aux signaux de contrôle du capteur d'image CMOS V10 et V11, ces signaux pouvant utiliser la norme I²C (pour *'Inter Integrated Circuit Bus)*
- au signal d'horloge du capteur d'image CMOS V12 ;
- aux signaux de transfert du flux vidéo V13, V14, V15 et V16, ces signaux pouvant utiliser la norme SubLVDS (pour 'Sub Low Voltage Differential Signalling') ;
- au signal de masse, V17.

De préférence, dans un mode de réalisation de la présente invention, le câblage qui relie la tête du dispositif d'exploration au terminal extérieur correspond à des micro-câbles.

En utilisant de tels micro-câbles et en mettant en oeuvre certaines connexions mécaniques de ces micro-câbles sur un support de circuit intégré destiné à recevoir les capteurs d'image, on peut optimiser la place occupée par les composants électroniques et par leurs connexions au niveau de la tête d'exploration. Dans ces conditions, on est en mesure de fournir une tête d'exploration de petite taille, alors même qu'elle est pourvue de deux capteurs d'image, de type CMOS par exemple, qui peut être introduite dans de petites cavités en étant relié vers l'extérieur par l'intermédiaire des micro-câbles.

## Revendications

1. Procédé de fabrication d'une tête (11) de dispositif d'exploration d'une cavité, ladite tête comprenant un support de circuit intégré présentant des première et seconde faces et une pluralité d'orifices traversants, à chaque orifice étant associées des première et seconde pastilles conductrices respectivement positionnées sur les première et seconde faces du support de circuit intégré :
ledit procédé comprenant les étapes suivantes mises en oeuvre pour chaque orifice dudit support de circuit intégré :
/a/ positionner (41) un micro-câble conducteur respectif dans l'orifice traversant, ce micro-câble présentant une partie dénudée sur une longueur supérieure ou égale à l'épaisseur du support ;
/b/ souder (42) ledit micro-câble sur les première et seconde pastilles conductrices associées ;
/c/ coller (43) le micro-câble avec une colle sur les première et seconde pastilles conductrices associées ; et
/d/ mouler (44) dans des première et seconde couches de résine le micro-câble respectivement sur les première et seconde pastilles conductrices, lesdites couches de résine couvrant complètement la partie dénudée dudit micro-câble.

2. Procédé de fabrication d'une tête selon la revendication 1, dans lequel le support de circuit intégré comprend un capteur d'image (21).

3. Procédé de fabrication d'une tête selon la revendication 2, dans lequel le capteur d'image (21) est de type CMOS.

4. Procédé de fabrication d'une tête selon l'une quelconque des revendications précédentes, dans lequel le support de circuit intégré comprend au moins une diode électroluminescente (22).

5. Procédé de fabrication d'une tête selon l'une quelconque des revendications précédentes, dans lequel le support de circuit intégré présente un orifice (23) adapté pour recevoir un outil d'intervention dans la cavité et/ou un orifice (24) adapté pour recevoir un canal d'acheminement d'eau.

6. Procédé de fabrication d'une tête selon l'une quelconque des revendications précédentes, ladite tête (11) présentant une première et une seconde face d'exploration et comprenant en outre un autre support de circuit intégré par application des étapes /a/ à /d/, lesdits supports de circuit intégré comprenant respectivement un premier et un second capteur d'image (601, 602) ;
ledit procédé de fabrication comprenant les étapes suivantes :
- disposer le support de circuit intégré du premier capteur d'image sur la première face d'exploration adaptée pour capter des images d'une première région de la cavité ;
- disposer le support de circuit intégré du second capteur d'image sur la seconde face d'exploration adaptée pour capter des images d'une seconde région de la cavité ;
lesdites première et seconde régions de la cavité étant distinctes l'une de l'autre.

7. Tête (11) de dispositif d'exploration d'une cavité, ladite tête comprenant un support de circuit intégré présentant des première et seconde faces et une pluralité d'orifices traversants, à chaque orifice étant associées des première et seconde pastilles conductrices (25) respectivement positionnées sur les première et seconde faces du support de circuit intégré, dans laquelle, à chaque orifice traversant dudit support de circuit intégré, correspond un micro-câble (13) présentant une partie dénudée sur une longueur supérieure ou égale à l'épaisseur dudit support, ledit micro-câble étant soudé et collé sur les première et seconde pastilles conductrices associées, et moulé dans des première et seconde couches de résine respectivement sur les première et seconde pastilles conductrices, lesdites couches de résine couvrant complètement la partie dénudée dudit micro-câble.

8. Tête (11) de dispositif d'exploration d'une cavité selon la revendication 7, présentant des première et seconde faces d'exploration et comprenant en outre un autre support de circuit intégré ;
lesdits supports de circuit intégré comprenant respectivement un premier et un second capteur d'image (601, 602) ;
le premier capteur d'image étant disposé sur la première face d'exploration et adapté pour capter des images d'une première région de la cavité ; et le second capteur d'image étant disposé sur la seconde face d'exploration et adapté pour capter des images d'une seconde région de la cavité ;
lesdites première et seconde régions de la cavité étant distinctes l'une de l'autre.

9. Tête (11) d'exploration selon la revendication 8, dans lequel la première face d'exploration correspond à une face latérale de ladite tête de dispositif d'exploration et la seconde face d'exploration correspond à une face avant de ladite tête.

10. Dispositif d'exploration d'une cavité comprenant :
une tête (11) de dispositif d'exploration d'une cavité selon l'une quelconque des revendications 7 à 9, et, d'autre part, comprenant au moins un capteur d'image; et
un module d'interface (12) adapté pour fournir des images captées par ledit capteur d'image, ledit module d'interface étant relié à la tête de dispositif d'exploration par lesdits micro-câbles (13).

11. Dispositif d'exploration d'une cavité selon la revendication 10, dans lequel la tête (11) présente un orifice (24) adapté pour recevoir un canal d'eau ; et un orifice (23) adapté pour recevoir un outil d'intervention dans la cavité.

12. Endoscope comprenant un dispositif d'exploration selon la revendication 10 ou 11.

## Claims

1. A method of manufacturing a head (11) of a cavity exploration device, the head including an integrated circuit support having first and second surfaces, and a plurality of through-holes each associated with respective first and second conducting pads respectively placed on the first and second surfaces of the integrated circuit support: the method comprising the following steps executed for each hole of the integrated circuit support:
/a/ positioning (41) respective conducting micro-cables in the through-holes, the micro-cables having an uninsulated portion of a length greater than or equal to a thickness of the support;
/b/ soldering (42) the micro-cables onto the first and second associated conducting pads;
/c/ gluing (43) the micro-cables with a glue onto the first and second associated conducting pads; and
/d/ molding (44) the micro-cable in first and second layers of resin onto the first and second conducting pads, respectively with said resin layers entirely covering the uninsulated portion of said micro-cable.

2. The method of manufacturing a head according to claim 1 wherein the integrated circuit support comprises an image sensor (21).

3. The method of manufacturing a head according to claim 2 wherein the image sensor is a CMOS sensor.

4. The method of manufacturing a head according to claim 1 wherein the integrated circuit support comprises at least one light-emitting diode.

5. The method of manufacturing a head according to anyone of the preceding claims wherein the integrated circuit support has a hole adapted to receive a tool for performing operations in the cavity and/or a hole (24) adapted to receive a tube for carrying water.

6. The method of manufacturing a head of anyone of the preceding claims wherein the head (11) comprises first and second exploration surfaces and further comprising another integrated circuit support in the head by implementation of the steps /a/ to /d/, the integrated circuit supports having a first and a second image sensor (601, 602) respectively, the integrated circuit support is a first integrated circuit support and the image sensor is a first image sensor, the method of manufacturing further comprising:
positioning the integrated circuit support of the first image sensor on the first exploration surface adapted to capture images of a first region of the cavity;
positioning the integrated circuit support of the second image sensor on the second exploration surface adapted to capture images of a second region of the cavity,
the first and second regions of the cavity being distinct one from the other.

7. A head (11) of a cavity exploration device, the head comprising an integrated circuit support having first and second surfaces and a plurality of through-holes each associated with corresponding first and second conducting pads (25) respectively placed on the first and second surfaces of the integrated circuit support, wherein each through-hole of the integrated circuit support corresponds to a micro-cable (13) having a portion which is uninsulated for a length greater than or equal to a thickness of the support, the micro-cable being soldered and glued to the first and second associated conducting pads, and molded in first and second layers of resin onto the first and second conducting pads, respectively, with said resin layers entirely covering the uninsulated portion of the micro-cable.

8. The head of a cavity exploration device according to claim 7 having first and second exploration surfaces and further comprising another integrated circuit support; said integrated circuit supports having a first and a second image sensor (601, 602) ;
the first image sensor being positioned on the first exploration surface and being adapted to capture images of a first region of the cavity ; and the second image sensor being positioned on the second exploration surface and being adapted to capture images of a second region of the cavity;
the first and second regions of the cavity being distinct one from the other.

9. The head of claim 8, wherein the first exploration surface corresponds to a lateral surface of the head of the exploration device and the second exploration surface corresponds to a front surface of said head.

10. A cavity exploration device comprising:
a head (10) of a cavity exploration device according to any one of the claims 7 to 9, and further comprising at least one image sensor; and,
an interface module (12) adapted to provide images captured by said image sensor, with said interface module being connected to the head of the exploration device by the micro-cables (13).

11. A cavity exploration device according to claim 10, wherein the head (11) has a hole (24) adapted to receive a tube of water, and a hole (23) adapted to receive a tool for performing operations in the cavity.

12. An endoscope comprising a cavity exploration device according to claim 10 or 11.

## Patentansprüche

1. Verfahren zur Herstellung eines Kopfs (11) einer Vorrichtung zur Untersuchung eines Hohlraums, wobei der Kopf einen Träger einer integrierten Schaltung umfasst, welcher erste und zweite Oberflächen und eine Mehrzahl an durchgängigen Öffnungen aufweist, wobei mit jeder der Öffnungen erste und zweite leitfähige Anschlussflächen (Lötaugen) verbunden sind, die jeweils auf den ersten und zweiten Oberflächen des Trägers der integrierten Schaltung angeordnet sind,
wobei das Verfahren folgende Schritte umfasst, die für jede Öffnung des Trägers der integrierten Schaltung durchgeführt werden:
/a/ Positionieren (41) eines leitfähigen Mikrokabels in jeder durchgängigen Öffnung, wobei dieses Mikrokabel einen blanken Abschnitt auf einer Länge, die größer oder gleich der Dicke des Trägers ist, aufweist;
/b/ Auflöten (42) des Mikrokabels auf die ersten und zweiten verbundenen leitfähigen Anschlussflächen;
/c/ Aufkleben (43) des Mikrokabels mit einem Klebstoff auf die ersten und zweiten verbundenen leitfähigen Anschlussflächen; und
/d/ Eingießen (44) des jeweiligen Mikrokabels auf den ersten und zweiten leitfähigen Anschlussflächen in erste und zweite Harzschichten, wobei die Harzschichten den blanken Abschnitt des Mikrokabels vollständig abdecken.

2. Verfahren zur Herstellung eines Kopfs gemäß Anspruch 1, wobei der Träger der integrierten Schaltung einen Bildaufnehmer (21) umfasst.

3. Verfahren zur Herstellung eines Kopfs gemäß Anspruch 2, wobei der Bildaufnehmer (21) vom CMOS-Typ ist.

4. Verfahren zur Herstellung eines Kopfs gemäß irgendeinem der vorhergehenden Ansprüche, wobei der Träger der integrierten Schaltung wenigstens eine Lumineszenz-Diode (22) umfasst.

5. Verfahren zur Herstellung eines Kopfs gemäß irgendeinem der vorhergehenden Ansprüche, wobei der Träger der integrierten Schaltung eine Öffnung (23), die zur Aufnahme eines Werkzeugs zum Eingreifen in den Hohlraum geeignet ist, und/oder eine Öffnung (24), die zur Aufnahme eines Kanals zur Beförderung von Wasser geeignet ist, aufweist.

6. Verfahren zur Herstellung eines Kopfs gemäß irgendeinem der vorhergehenden Ansprüche, wobei der Kopf (11) eine erste und eine zweite Abtastoberfläche aufweist und außerdem einen weiteren Träger einer integrierten Schaltung durch Ausführen der Schritte /a/ bis /d/ umfasst, wobei die Träger der integrierten Schaltungen jeweils einen ersten und einen zweiten Bildaufnehmer (601, 602) umfassen;
wobei das Herstellungsverfahren die folgenden Schritte umfasst:
- Anordnen des Trägers der integrierten Schaltung des ersten Bildaufnehmers auf der ersten Abtastoberfläche, die zur Aufnahme von Bildern von einem ersten Bereich des Hohlraums geeignet ist;
- Anordnen des Trägers der integrierten Schaltung des zweiten Bildaufnehmers auf der zweiten Abtastoberfläche, die zur Aufnahme von Bildern von einem zweiten Bereich des Hohlraums geeignet ist;
wobei die ersten und zweiten Bereiche des Hohlraums voneinander verschieden sind.

7. Kopf (11) einer Vorrichtung zur Untersuchung eines Hohlraums, wobei der Kopf einen Träger einer integrierten Schaltung umfasst, welcher erste und zweite Oberflächen und eine Mehrzahl an durchgängigen Öffnungen aufweist, wobei mit jeder der Öffnungen erste und zweite leitfähige Anschlussflächen (25) verbunden sind, die jeweils auf den ersten und zweiten Oberflächen des Trägers der integrierten Schaltung angeordnet sind, wobei jeder durchgängigen Öffnung des Trägers der integrierten Schaltung ein Mikrokabel (13) entspricht, welches einen blanken Abschnitt auf einer Länge, die größer oder gleich der Dicke des Trägers ist, aufweist, wobei das Mikrokabel auf die ersten und zweiten verbundenen leitfähigen Anschlussflächen gelötet und geklebt und in erste und zweite Harzschichten jeweils auf den ersten und zweiten leitfähigen Anschlussflächen eingegossen ist, wobei die Harzschichten den blanken Abschnitt des Mikrokabels vollständig abdecken.

8. Kopf (11) einer Vorrichtung zur Untersuchung eines Hohlraums gemäß Anspruch 7, welcher erste und zweite Abtastoberflächen aufweist und außerdem einen weiteren Träger einer integrierten Schaltung umfasst;
wobei die Träger der integrierten Schaltungen jeweils einen ersten und einen zweiten Bildaufnehmer (601, 602) umfassen;
wobei der erste Bildaufnehmer auf der ersten Abtastoberfläche angeordnet ist und zur Aufnahme von Bildern von einem ersten Bereich des Hohlraums geeignet ist; und
wobei der zweite Bildaufnehmer auf der zweiten Abtastoberfläche angeordnet ist und zur Aufnahme von Bildern von einem zweiten Bereich des Hohlraums geeignet ist;
wobei die ersten und zweiten Bereiche des Hohlraums voneinander verschieden sind.

9. Untersuchungskopf (11) gemäß Anspruch 8, wobei die erste Abtastoberfläche einer lateralen Oberfläche des Kopfs der Untersuchungsvorrichtung entspricht, und die zweite Abtastoberfläche einer Oberfläche vor dem besagten Kopf entspricht.

10. Vorrichtung zur Untersuchung eines Hohlraums, umfassend:
einen Kopf (11) einer Vorrichtung zur Untersuchung eines Hohlraums gemäß irgendeinem der Ansprüche 7 bis 9, welcher seinerseits wenigstens einen Bildaufnehmer umfasst; und
ein Schnittstellen-Modul (12), welches geeignet ist, Bilder zu liefern, die durch den Bildaufnehmer aufgenommen werden, wobei das Schnittstellen-Modul mit dem Kopf der Untersuchungsvorrichtung durch Mikrokabel (13) verbunden ist.

11. Vorrichtung zur Untersuchung eines Hohlraums gemäß Anspruch 10, wobei der Kopf (11) eine Öffnung (24), die zur Aufnahme eines Wasserkanals geeignet ist, und eine Öffnung (23), die zur Aufnahme eines in den Hohlraum eingreifenden Werkzeugs geeignet ist, aufweist.

12. Endoskop, welches eine Untersuchungsvorrichtung gemäß Anspruch 10 oder 11 umfasst.
